# FASCICULE DE BREVET EUROPEEN

(11) **EP 1 091 966 B1**
(45) Date de publication et mention de la délivrance du brevet: **06.11.2002**
(21) Numéro de dépôt: 99926580.4
(22) Date de dépôt: 01.07.1999
(51) Int. Cl.: C07F 9/30, A61K 31/66, C07F 9/572

(54) **INHIBITEURS SELECTIFS DU SITE N-TERMINAL DE L' ECA**
SELEKTIVE INHIBITOREN FÜR DEN N-TERMINALEN SITE DER ACE
N-TERMINAL SITE SELECTIVE INHIBITORS OF HUMAN ANGIOTENSIN CONVERSION ENZYME (ACE)

(30) Priorité: 02.07.1998 FR 9808464
(43) Date de publication de la demande: 18.04.2001
(73) Titulaire: Commisariat à l'énergie Atomique, 75752 Paris 15ème (FR); INSTITUT NATIONAL DE LA SANTE ET DE LA RECHERCHE MEDICALE (INSERM), 75654 Paris Cédex 13 (FR)
(72) Inventeur: DIVE, Vincent, F-94160 Saint Mande (FR); COTTON, Joel, F-91400 Orsay (FR); CUNIASSE, Philippe, F-75020 Paris (FR); YIOTAKIS, Athanasios, GR-153 42 Athenes (GR); CORVOL, Pierre, F-75007 Paris (FR); MICHAUD, Annie, F-93100 Montreuil (FR); CHAUVET, Marie-Thérèse, F-92310 Sèvres (FR); MENARD, Joel, F-75013 Paris (FR); EZAN, Eric, F-92240 Malakof (FR)
(74) Mandataire: Des Termes, Monique
(86) Numéro de dépôt international: FR9901581
(87) Numéro de publication internationale: WO00001706

(56) Documents cités:
- EP-A- 0 209 848
- EP-A- 0 725 075
- WO-A-98/18803
- FR-A- 2 676 059

## Description

### Domaine technique

La présente invention a pour objet un dérivé de peptide phosphinique, capable de bloquer sélectivement le site N-terminal de l'enzyme de conversion de l'angiotensine humaine (ECA) sans affecter le deuxième site actif de l'ECA.

Ces dérivés de peptide qui sont des inhibiteurs sélectifs du site N-terminal de l'ECA peuvent être utilisés en thérapeutique pour protéger les cellules souches hématopoïétiques de patients soumis à une chimiothérapie ou une radiothérapie agressive.

### Etat de la technique antérieure

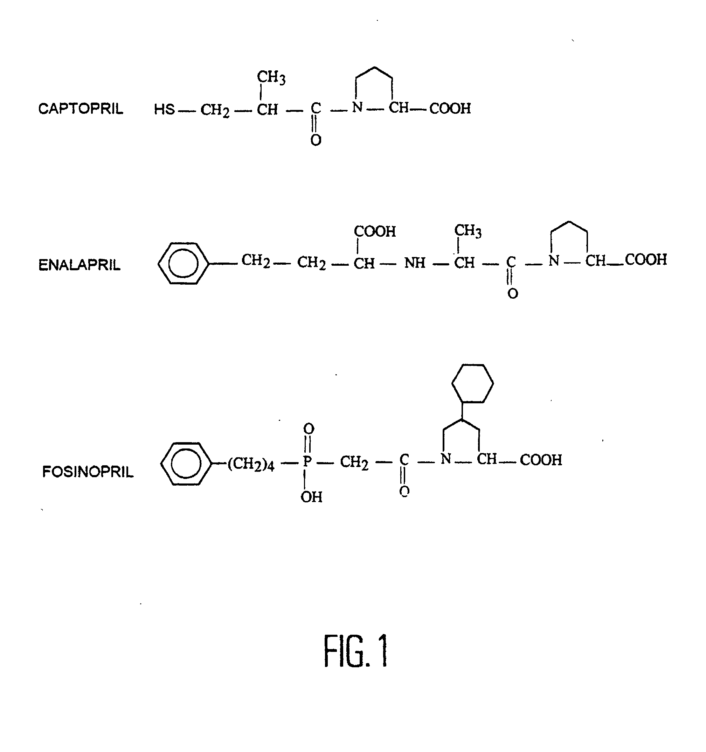

Le développement d'inhibiteurs pseudo-peptidiques de l'ECA a, dans les années 80, véritablement révolutionné le traitement de l'hypertension artérielle, de l'insuffisance cardiaque et de l'insuffisance rénale chronique. Ainsi, il existe aujourd'hui toute une panoplie d'inhibiteurs synthétiques de l'ECA qui sont utilisés en clinique. Parmi ceux-ci, on connaît le captopril, l'énalapril et le fosinopril dont les formules sont données sur la figure 1.

Parallèlement à ces travaux, grâce au clonage de l'enzyme ECA en 1988, le groupe de P. Corvol a pu démontrer l'existence de deux sites actifs dans cette enzyme, comme il est décrit par Soubrier et al dans Proc. Natl. Acad. Sci. USA (1988) 85, 9386-9390 [1].

La démonstration formelle que ces deux sites actifs de l'ECA contrôlent chez l'homme des fonctions physiologiques distinctes, constitue une autre révolution dans ce domaine et a des conséquences importantes en termes thérapeutiques. Dans la mesure où tous les inhibiteurs de l'ECA mis au point à ce jour inhibent *in vitro* à un degré similaire les deux sites actifs de l'ECA, la fonction physiologique de ces deux sites actifs in vivo ne peut être abordée avec ce type de composés. Les premiers inhibiteurs capables de discriminer les deux sites actifs de l'ECA seraient en revanche extrêmement précieux.

L'ECA hydrolyse plusieurs substrats naturels impliqués dans la régulation de la pression artérielle, du volume sanguin circulant et de l'hémodynamique cardiovasculaire. Le principal substrat est l'angiotensine I, décapeptide inactif, qui est activé en angiotensine II, peptide vasopresseur et antinatriurétique, après hydrolyse du dipeptide carboxyterminal His-Leu. Parallèlement, l'ECA inactive la bradykinine, peptide vasodilatateur et natriurétique en un heptapeptide puis en un pentapeptide, tous les deux inactifs. Les deux sites N- et C-terminaux de l'ECA sont impliqués de façon similaire dans cette hydrolyse.

Une fonction spécifique du domaine N-terminal de l'ECA a été récemment identifiée par le groupe de P. Corvol. Comme il est décrit par Lenfant et al dans Proc. Natl. Acad. Sci. USA (1989) 86, 779-782 [2], le peptide N-Acétyl-Séryl-Aspartyl-Lysyl-Proline (AcSDKP) est un inhibiteur circulant naturel de l'entrée en phase S des cellules souches hématopoïétiques. Il bloque aussi l'entrée en phase S d'autres types cellulaires tels que les hépatocytes en phase de régénération, les lymphocytes et plusieurs lignées cellulaires établies, comme il est décrit par Lombard et al, dans Cell. Tissue. Kinet (1990) 23, 99-103 [3]. L'action inhibitrice de l'AcSDKP sur le cycle cellulaire des cellules hématopoïétiques est spécifique des cellules hématopoïétiques normales ; les cellules leucémiques ne sont pas concernées. L'AcSDKP a donc été proposé comme agent thérapeutique pour la protection des progéniteurs médullaires au cours de chimiothérapies. De fait, l'administration d'AcSDKP prolonge la survie de souris traitées par des agents cytotoxiques (Bodgen et al, Ann. N.Y. Acad. Sci. (1991) 628, 126-139 [4]). Comme il est décrit par Rousseau et al dans J. Biol. Chem. (1995) 270, 3656-3661 [5] et Azizi et al dans J. Clin. Invest. (1996) 97, 839-844 [6], l'AcSDKP est hydrolysé in vitro et in vivo par l'ECA, et plus particulièrement par le domaine N-terminal de l'enzyme. In vitro, l'AcSDKP est hydrolysé 50 fois plus rapidement par le domaine N-terminal que par le domaine C-terminal. Cette découverte montre qu'il serait possible de développer des inhibiteurs spécifiques du domaine N- ou C-terminal de l'ECA, permettant d'agir sur des substrats impliqués dans d'autres fonctions que la régulation de la pression artérielle et du métabolisme hydrosodé.

L'ECA est l'enzyme principale, voir exclusive, du métabolisme de l'AcSDKP plasmatique. L'administration d'une dose unique de captopril chez des sujets sains élève de 6 à 7 fois le taux plasmatique du peptide. Un inhibiteur sélectif du domaine N-terminal permettrait d'obtenir un tel résultat sans modifier, à l'inverse du captopril et des autres inhibiteurs de l'enzyme de conversion utilisés à ce jour, le métabolisme des peptides jouant un rôle dans le contrôle de la pression artérielle et du métabolisme hydrosodé (angiotensine, bradykinine). Des inhibiteurs du domaine N-terminal de l'ECA seraient donc d'un grand intérêt pour la protection des cellules souches hématopoïétiques des patients soumis à un traitement chimiothérapique ou radiothérapique agressif. L'inhibiteur pourrait être administré avant ou concomitamment à la thérapeutique anti-cancéreuse.

Il a par ailleurs été montré récemment, par Volpert et al dans J. Clin. Invest. (1996) 98, 671-679 [7] que le captopril, qui inhibe les deux sites actifs de l'ECA, pourrait exercer expérimentalement un effet protecteur, anti-cancéreux, in vitro et in vivo. Le mécanisme de cet effet protecteur n'est pas connu mais fait sans doute intervenir l'AcSDKP, du fait de ses propriétés sur l'entrée dans le cycle cellulaire de nombreux types de cellules. Un inhibiteur sélectif du domaine N-terminal de l'ECA, potentialisant le taux plasmatique de l'AcSDKP, sans modifier le métabolisme des peptides vasoactifs, pourrait donc avoir un effet bénéfique.

La plupart des inhibiteurs puissants de l'ECA développés à ce jour et illustrés sur la figure 1 se caractérisent par la présence d'un corps pseudo-dipeptidique sur lequel a été greffé un groupement chimique capable d'interagir favorablement avec l'atome de zinc situé dans le site actif de l'ECA. En effet, l'ECA appartient au groupe des métalloprotéases à zinc et se caractérise par la présence d'un atome de zinc dans les deux sites actifs de l'enzyme, atome de zinc jouant un rôle essentiel dans l'acte catalytique. De nombreux travaux ont pu démontrer que la synthèse de pseudo-peptides comportant des groupements chimiques capables de chélater le zinc fournissait une voix d'accès à des inhibiteurs très puissants des métalloprotéases à zinc. Parmi les groupements chimiques capables d'interagir avec le zinc, on retrouve dans les inhibiteurs commerciaux de l'ECA, le groupe thiol HS (captopril), le groupe carboxyalkyle CH-COO (énalapril), et le groupe phosphoryle PO₂-X avec X=NH,O,CH₂ (Fosinopril).

Les documents FR-A-2 676 059 [8] et EP-A-0 725 075 [9] illustrent des dérivés de peptides utilisables comme inhibiteurs de protéases à zinc, qui comportent des groupements de type phosphinique pour interagir avec le zinc. Dans FR-A-2 676 059, il s'agit d'inhibiteurs des collagénases bactériennes alors que dans EP-A-0 725 075, il s'agit d'inhibiteurs sélectifs de l'endopeptidase à zinc 24-15, qui sont inactifs vis-à-vis d'autres protéases telles que l'enzyme de conversion de l'angiotensine.

Le document WO-A-98/18803 décrit des dérivés d'(α-aminophosphino)peptide répondant à la formule : 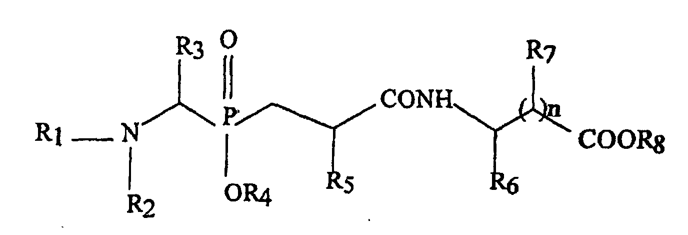 dans laquelle R₁ et R₂ représentent un atome d'hydrogène ou forment ensemble une imine avec l'atome d'azote adjacent et R₃, R₄, R₅, R₆, R₇ et R₈ représentent indépendamment un atome d'hydrogène ou des groupes hydrocarbonés.

Ces dérivés sont des inhibiteurs mixtes de l'endopeptidase neutre et de l'aminopeptidase N.

Le document EP-A-0 209 848 décrit des peptides inhibiteurs de l'enzyme de conversion de l'angiotensine humaine, de formule A-B-D-E-G dans laquelle E peut répondre à la formule :

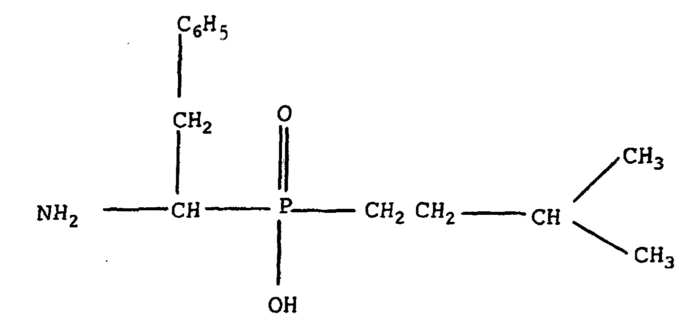

Ainsi, jusqu'à présent, il n'existait pas d'inhibiteurs sélectifs du site N-terminal de l'enzyme de conversion de l'angiotensine.

La présente invention a précisément pour objet de nouveaux dérivés de peptides comportant un groupement phosphinique, qui sont des inhibiteurs sélectifs du site N-terminal de cette enzyme.

### Exposé de l'invention

Selon l'invention, ces nouveaux dérivés de peptides comportent la séquence d'acides aminés répondant à la formule suivante :

Asp-Phe-Ψ(PO₂CH₂)-Ala-Xaa'- (I)

dans laquelle :
- Ψ(PO₂CH₂) indique que la liaison peptidique (CONH) entre Phe et Ala a été remplacée par la liaison phosphinique PO₂CH₂, et
- Xaa' représente un résidu d'acide aminé.

Dans cette séquence, le groupe PO₂CH₂ est sous forme PO₂⁻ ; il est donc associé à un contre-ion tel que K⁺, Na⁺ ou tout autre métal acceptable du point de vue pharmacologique. La nature du contre-ion n'a aucune importance car dans l'eau les groupements chargés sont dissociés.

Selon un mode particulier de réalisation de l'invention, le dérivé de peptide comporte uniquement les quatre acides aminés de cette séquence et répond à la formule suivante :

R¹-Asp-Phe-Ψ(PO₂CH₂)-Ala-Xaa'-NH₂ (II)

dans laquelle :
- R¹ représente le groupe acétyle ou benzyloxycarbonyle,
- Ψ(PO₂CH₂) indique que la liaison peptidique (CONH) entre Phe et Ala a été remplacée par la liaison phosphinique PO₂CH₂, et
- Xaa' représente un résidu d'acide aminé.

De préférence R¹ représente le groupe acétyle.

Dans les formules (I) et (II) données ci-dessus, les acides aminés utilisés pour Xaa' peuvent être des acides aminés naturels ou non-naturels, ou des pseudo- acides aminés.

Les acides aminés naturels peuvent être choisis parmi l'alanine, l'arginine, l'asparagine l'acide aspartique, la cystéine, la glutamine, l'acide glutamique, la glycine, l'histidine, l'isoleucine, la leucine, la norleucine, la lysine, la méthionine, la phénylalanine, la proline, l'hydroxyproline, la sérine, la thréonine, le tryptophane, la tyrosine, la valine, la nitrophénylalanine, l'homoarginine, la thiazolidine et la déshydropoline.

Un pseudo-acide aminé peut être défini comme un acide aminé dans lequel la fonction amino ou carbonyle a été remplacée par un autre groupement chimique.

Dans ces formules, les acides aminés Asp, Phe, Ala et Xaa' peuvent être sous forme L ou D. Le dérivé de peptide peut donc être constitué par un seul isomère ou par un mélange de 4 diastéréoisomères, dus à la présence de deux centres asymétriques dans le dérivé.

Dans le dérivé de peptide de l'invention, l'acide aminé Xaa' est choisi de préférence parmi les acides aminés suivants : Pro, Ala, Thr, Lys et Leu qui ont une activité faible vis-à-vis du site C-terminal de l'ECA.

De préférence, Xaa' représente Ala car cet acide aminé renforce l'activité du dérivé de peptide pour l'inhibition du site N-terminal de l'enzyme en ayant une activité d'inhibition faible pour le site C-terminal.

Les dérivés de peptides de l'invention sont différents des dérivés de peptides décrits dans EP-A-0 725 075 car ils comportent avant la pseudo-Phe un résidu Asp qui permet de rendre inactif le dérivé de peptide vis-à-vis du site C-terminal de l'enzyme de conversion de l'angiotensine et confère ainsi la sélectivité voulue. D'ailleurs, les dérivés de peptides du EP-A-0 725 075 étaient inactifs vis-à-vis de cette enzyme alors que les dérivés de l'invention sont actifs vis-à-vis de la partie N-terminale de cette enzyme.

La présence des résidus Asp et pseudo-Phe permet aux dérivés de peptides d'agir sur les sous-sites S1 et S2 de l'enzyme, ce qui est une propriété peu usuelle pour un inhibiteur de ECA. Le fait encore plus surprenant est que ce dérivé de peptide comporte en position C-terminale de sa structure, un groupe carboxamide, alors que tous les inhibiteurs de ECA décrits à ce jour possèdent systématiquement un groupe carboxylate libre. Ainsi, ce dérivé de peptide apparaît très original tant du point de vue de sa structure chimique que de son activité inhibitrice.

Selon l'invention, bien que toute configuration des acides aminés Phe et Ala puisse convenir, il est préférable que Phe ait la configuration R. Par ailleurs, il est préférable également que le résidu d'acide aminé Ala ait la configuration S. Ainsi, le dérivé de peptide préféré répond à la formule :

Ac-Asp-_{(R)}Phe-Ψ(PO₂CH₂)-_{(S)}Ala-Ala-NH₂ (III)

dans laquelle :
- Ac représente le groupe acétyle, et
- Ψ(PO₂CH₂) indique que la liaison peptidique (CONH) entre Phe et Ala a été remplacée par la liaison phosphinique (PO₂CH₂).

Les dérivés de peptides de l'invention peuvent être préparés par des procédés classiques tels que celui décrit dans FR-A-2 676 059 ou encore par un procédé de synthèse en phase solide tel que celui décrit dans EP-A-0 725 075 en partant du synthon de formule :

Fmoc-PheΨ(PO (Ad)-CH₂)AlaOH

où Fmoc représente le groupe (fluorénylinéthoxy)carbonyle et Ad représente le groupe adamantyle, et en utilisant comme support solide la résine 2-chlorotrityle.

Ce synthon peut être préparé en particulier en suivant le protocole décrit par Yiotakis et al dans J. Org. Chem., 1996, 61, 6601-6605 [10].

L'invention a encore pour objet une composition pharmaceutique inhibant sélectivement le site N-terminal de l'enzyme de conversion de l'angiotensine humaine, qui comprend un dérivé de peptide répondant à la formule (I), (II) ou (III) données ci-dessus.

Cette composition pharmaceutique est utilisable en particulier pour protéger les cellules souches hématopoïétiques de patients soumis à un traitement chimiothérapique ou radiothérapique agressif, par exemple un traitement anti-cancéreux.

L'invention concerne encore l'utilisation d'un dérivé de peptide de formule (I), (II) ou (III) décrites ci-dessus pour la fabrication d'un médicament inhibant sélectivement le site N-terminal de l'enzyme de conversion de l'angiotensine humaine.

Un tel médicament peut être destiné à réguler la prolifération des cellules souches hématopoïétiques de patients soumis à un traitement anti-cancéreux.

D'autres caractéristiques et avantages de l'invention apparaîtront mieux à la lecture de la description qui suit, donnée bien entendu à titre illustratif et non limitatif, en référence aux dessins annexés.

### Brève description des dessins.

La figure 1 déjà décrite illustre la structure d'inhibiteurs connus de l'enzyme de conversion de l'angiotensine humaine, conformes à l'art antérieur.

La figure 2 illustre les pourcentages d'inhibition vis-à-vis du site C-terminal et du site N-terminal de l'enzyme de conversion de l'angiotensine humaine, de différents mélanges de peptides.

La figure 3 illustre les pourcentages d'inhibition vis-à-vis du site C-terminal et du site N-terminal de divers dérivés de peptides.

La figure 4 est un chromatogramme obtenu par HPLC en phase inverse d'un mélange de diastéréoisomères du dérivé de peptide Ac-Asp-PheΨ(PO₂CH₂)Ala-Ala-NH₂ conforme à l'invention.

La figure 5 est un chromatogramme HPLC en phase inverse du mélange de deux diastéroisomères du dérivé de peptide Ac-Asp-PheΨ(PO₂CH₂)Ala-Ala-NH₂ conforme à l'invention.

La figure 6 illustre le pourcentage d'inhibition obtenu par le dérivé de peptide RXP 407 en fonction de la concentration utilisée.

La figure 7 illustre l'évolution en fonction du temps de la concentration plasmatique d'un dérivé de peptide de l'invention chez le rat.

La figure 8 illustre l'évolution en fonction du temps de la concentration plasmatique en peptide Ac-Ser-Asp-Lys-Pro de souris perfusées avec l'inhibiteur de l'invention RXP 407 à des doses de 0,1, 1 et 10 mg/kg (courbes 4, 5 et 6), de souris perfusées avec du Lisinopril à une dose de 10 mg/kg (courbe 7) et des souris témoins non perfusées (courbe T).

La figure 9 illustre l'évolution en fonction du temps de la concentration plasmatique en angiotensine I (ng/ml/h) de souris perfusées avec RXP 407 à des doses de 0,1, 1 et 10 mg/kg (courbes 8, 9 et 10), de souris perfusées avec 10 mg/kg de Lisonopril (courbe 11), ainsi que de souris non perfusées (courbe T).

### Exposé détaillé des modes de réalisation

### Exemple 1

Dans cet exemple, on a synthétisé vingt mélanges de formule générale Ac-Xaa-PheΨ(PO₂CH₂)Ala-Xaa'-NH_{2,}, dans lesquels la position Xaa est occupée par un acide aminé de structure connue, alors que dans la position Xaa' les vingt acides aminés naturels sont représentés de façon équimoléculaire.

On a utilisé pour cette synthèse une approche de chimie combinatoire décrite par Jiracek et al dans J. Biol. Chem. (1995) 270, 21701-21706, [12] et Jiracek et al, J. Biol Chem. (1996) 271, 19606-19661, [13], et une synthèse en phase solide en utilisant comme bloc phosphinique le synthon Fmoc-PheΨ(FO(Ad)CH₂)AlaOH, obtenu selon la procédure décrite par Yiotakis et al dans J. Org. Chem (1996) 61, 6601-6605, [10] pour les synthons de type 5.

### a) Synthèse du bloc phosphinique Fmoc-Phe(PO(Ad)-CH₂)AlaOH.

### 1. Préparation du bloc Z-Phe(PO₂-CH₂)AlaOet (Z = benzyloxycarbonyle).

Une suspension de Z-Phe(PO₂)H (1mmol) et d'hexaméthydisilazane (5mmol) est chauffée à 110°C, sous une atmosphère d'argon pendant 1 heure. Après refroidissement à 90°C, de l'éthyl méthylacrylate (1,3 mmol) est ajouté goutte à goutte pendant 30 minutes. La réaction est laissée pendant 3 heures, sous agitation, à 90°C. Après avoir ramené le mélange réactionnel à la température de 70°C, on ajoute 3 ml d'éthanol absolu goutte à goutte. Après retour à la température ambiante, les produits volatils sont éliminés par évaporation sous vide, et le produit restant repris dans NaHCO₃ 10 %. Cette phase aqueuse est rincée à l'éther, acidifiée à pH 1,5 avec HCl 1N, puis le précipité est repris dans l'acétate d'éthyle. La phase organique est séchée avec du Na₂SO₄, évaporée à sec pour donner le bloc Z-Phe(PO₂-CH₂)AlaOet avec un bon rendement.

### 2) Préparation du bloc Z-Phe(PO(Ad)-CH₂)AlaOet.

Le composé Z-Phe(PO₂-CH₂)AlaOet (1 mmol) est dissous dans l'éthanol 95 % (25 mL). Cette solution est ajoutée, goutte à goutte, à une solution 0,5 M de nitrate d'argent. Après 10 minutes, 15 mL d'eau sont ajoutés au mélange réactionnel, et l'éthanol est évaporé sous vide. La phase aqueuse restante, contenant le précipité d'argent, est refroidie avec un bain d'eau et de glace. Le précipité formé est filtré, lavé à l'eau froide, et séché sous vide en présence de P₂O₅, pour donner un sel d'argent du bloc phosphinique avec un rendement de 90 %. Ce produit (1 mmol) est additionné à une solution de bromure d'adamantyle (1,2 mmol) dans le chloroforme (10mL). Ce mélange est porté à reflux pendant 30 minutes. Le bromure d'argent, qui précipite, est éliminé par filtration, et le mélange réactionnel restant est évaporé à sec sous vide. Le produit attendu est purifié par flash chromatographie (éluant : chloroforme/isopropanol, 9 : 3)avec un rendement de 80 %.

### 3) Préparation du bloc Z-Phe(PO(Ad)-CH₂)AlaOH.

Le bloc Z-Phe(PO(Ad)-CH₂)AlaOet (1 mmol) est disous dans l'éthanol (10 mL). A cette solution est ajoutée 1 mL de soude 4N, goutte à goutte. Après 2 heures de réaction, l'éthanol est évaporé sous vide, et le résidu dilué dans 20 mL d'eau. Le mélange réactionnel est refroidi par un bain de glace, puis acidifié à pH 2 avec HCl 0,5 N. Le produit qui précipite est repris dans l'éther, la phase organique est rincée à l'eau, séchée avec du NaSO₄, puis évaporée à sec pour donner le bloc phosphinique saponifié, avec un rendement de 95 %.

### 4) Préparation du bloc Fmoc-Phe(PO(Ad)-CH₂)AlaOH.

A une solution de méthanol (RmL), contenant le bloc phosphinique Z-Phe(PO(Ad-)CH₂)AlaOH (1 mmol) et du formiate d'ammonium (4 mmol), est ajouté 0,25 g de Pd/C 10 %. Après 12 minutes à température ambiante, le catalyseur est éliminé par filtration sur célite, puis le résidu est séché sous vide à sec. Ce résidu est repris dans du dichlorométhane, puis évaporé à sec. Cette procédure est répétée plusieurs fois. Ce résidu est traité avec du chloroforme et l'excès de formiate d'ammonium n'ayant pas réagi et présent sous forme de précipité, est éliminé par filtration. Le produit formé est repris dans Na₂CO₃ (3mL). Le mélange réactionnel est évaporé à moitié sous vide, puis sont rajoutés 1,5 mL d'eau et 2 mL de dioxane. Une solution de Fmoc-Cl (1,2 mmol) dans le dioxane (2 mL) est ajouté au mélange réactionnel, refroidi par un bain de glace. Cette solution est laissée sous agitation pendant 2 heures à 4°C, puis 4 heures à la température ambiante. Le mélange réactionnel est dilué avec 20 mL d'eau, refroidi par un bain de glace et acidifié à pH 2 avec HCl 2N. Le produit qui précipite est repris rapidement dans l'éther. Cette phase organique est rincée à l'eau, séchée avec Na₂SO₄, puis évaporée sous vide à sec pour donner le produit désiré, qui est purifié par flash chromatographie (chloroforme/méthanol, 9,5 : 0,5) avec un rendement final de 65 %.

### b) Synthèse des peptides phosphiniques.

Pour la synthèse des peptides phosphiniques, le premier amino-acide Xaa est attaché à la résine 2-chlorotrityle en utilisant la technique de Barlos et al décrite dans G. Int. J. Pept. Protein., Res, 1991, 37, 513-520 [11]. On fixe ensuite sur ce premier amino acide le bloc phosphinique, puis les acides aminés Xaa'. On élimine le groupe Fmoc avec de la pipéridine à 30 % dans de la N-méthylpyrrolidone et on couple les résidus suivants Xaa' en utilisant le mélange 2-(1H)benzotriazol-1-yl)-1,1,3,3-tétraméthyluronium hexafluorophosphate/diisopropyl éthyl amine. Dans ce procédé; les différentes étapes sont réalisées par des techniques classiques en utilisant les réactifs et solvants généralement employés dans la chimie des peptides, comme il est décrit dans Yiotakis et al, J. Org. Chem (1996) 61, 6601-6605, [10].

On obtient ainsi des mélanges de peptides comportant 20 acides aminés différents en position Xaa et 20 acides aminés différents en position Xaa', les mélanges de peptides étant séparés et identifiés selon la nature de l'acide aminé en position Xaa.

Ces mélanges, ont été évalués à des concentrations finales en peptide de 200 nM afin de déterminer leur capacité à inhiber l'ECA. Pour doser l'ECA, on a développé un nouveau substrat à fluorescence quenchée de l'ECA (Mca-Ala-Ser-Asp-Lys-Dpa) avec Mca représentant la méthoxy coumarine et Dpa l'acide 2-(N-dinitrophényl)aminopropionique. Ce substrat, qui ne fluoresce pas lorsqu'il est intact, émet un signal de fluorescence intense après clivage, entre les résidus Asp et Lys, par l'ECA. Grâce à ce type de substrat, on peut tester le pouvoir inhibiteur d'un très grand nombre de composés dans des plaques Elisa.

Le pouvoir inhibiteur des composés est déterminé dans un test de compétition classique basé sur la dégradation du substrat à fluorescence quenchée Mca-Ala-Asp-Ser-Lys-Pro. La dégradation de ce substrat par l'ECA conduit à l'observation d'un signal de fluorescence à 400 nm, lorsque l'échantillon est excité à 328 nm. Les expériences d'inhibition sont réalisées dans des puits de plaques Elisa, dans des volumes de 200 µL du tampon : pH 6,8, 50 mM Hepes, 200 mM NaCl, 1 ZnCl₂, à 25°C, dans un appareil du type Fluorolite 1000 de la société Dynatech. La concentration du substrat dans une expérience type est de 20 µM. Pour les expériences d'inhibition, l'inhibiteur est mis en présence de l'enzyme pendant 45 min, puis la réaction est initialisée par simple addition d'un très faible volume de substrat. Les pourcentages d'inhibition sont calculés à partir des variations de vitesses initiales en présence d'inhibiteur. Les cinétiques de dégradation ont été déterminées à partir de l'enregistrement des variations de fluorescence sur une gamme de temps de 40 min.

L'activité inhibitrice de chaque mélange a été évaluée sur deux formes mutantes de l'ECA: la forme dite N-terminale, dans laquelle le site C-terminal est rendu inactif par mutagénèse et la forme C-terminale comportant un site N-terminal inactif, comme il est décrit par Wei et al, dans J. Biol. Chem. (1992) 267, 13398-13405, [14]. Ces mutants ont été produits à partir de cellules CHO.

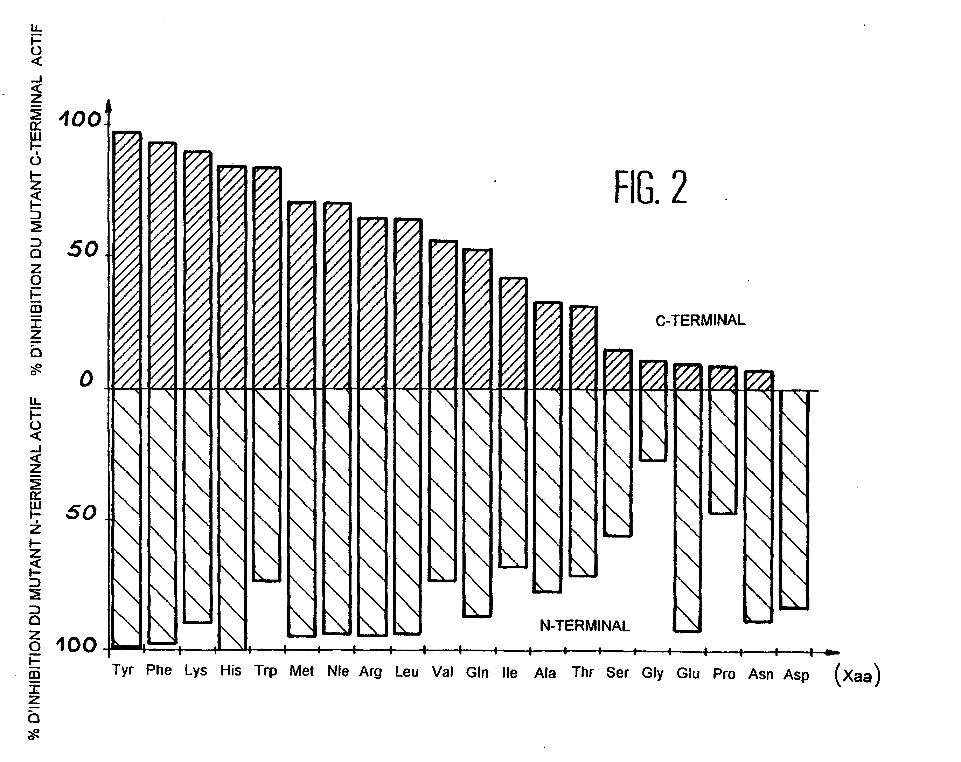

Sur la figure 2, on a indiqué les pourcentages d'inhibition observés avec les mutants Net C-terminaux de l'ECA en fonction de la nature de l'acide aminé présent dans la position Xaa des inhibiteurs. Cette figure démontre qu'un très grand nombre de substitutions sont compatibles pour l'inhibition aussi bien du site N-terminal que C-terminal de l'ECA. En terme de sélectivité, on remarque cependant que seule la présence d'un résidu aspartique en position Xaa permet d'inhiber le site N-terminal, mais en revanche rend inactif le mélange d'inhibiteurs comportant ce résidu vis-à-vis du site C-terminal de l'ECA.

Ces résultats montrent ainsi qu'il est essentiel d'avoir un résidu Asp en position Xaa pour obtenir un inhibiteur sélectif du site N-terminal.

### Exemple 2

Sur la base des résultats de l'exemple 1, on a synthétisé, selon le même mode opératoire que dans l'exemple 1, vingt peptides phosphiniques de formule générale, Ac-Asp-PheΨ(PO₂CH₂)Ala-Xaa'-NH₂, dans lesquels la position Xaa' est occupée par un seul acide aminé. On a testé ces peptides comme dans l'exemple 1 pour déterminer leur activité inhibitrice sur les sites N-terminal et C-terminal de l'ECA.

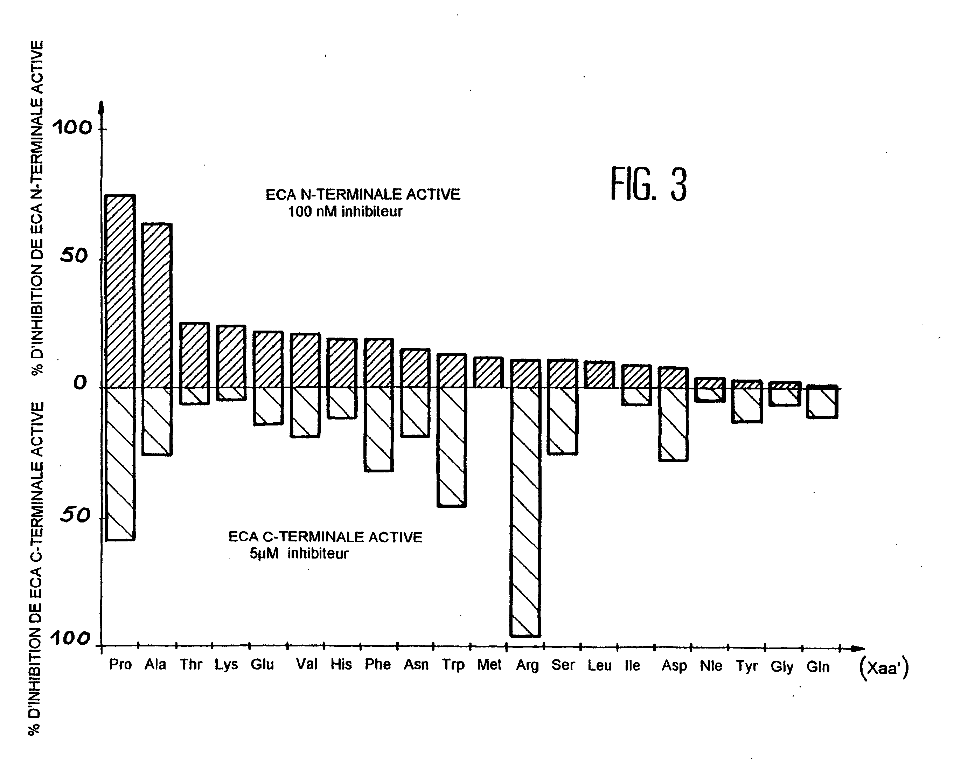

Les résultats obtenus sont représentés sur la figure 3 qui illustre les pourcentages d'inhibition de ces peptides sur les sites N et C-terminaux de l'ECA en fonction de la nature du résidu en position Xaa'.

Il convient de remarquer que pour le site N-terminal les inhibiteurs ont été étudiés à une concentration de 100 nM, alors que pour le site C-terminal la concentration utilisée pour observer une inhibition notable a été fixée à 5 µM. Cette différence de concentration reflète la meilleure affinité de ces composés pour le site N-terminal de l'ECA.

Sur la figure 3, on constate que la présence dans la position Xaa' des peptides des résidus proline et alanine permet d'améliorer le degré de sélectivité en faveur du site N-terminal et que les résidus Thr, Lys, Met et Leu sont très peu actifs sur le site C-terminal de l'enzyme.

### Exemple 3

Dans cet exemple, on a déterminé les constantes d'inhibition Ki du composé Ac-Asp-PheΨ(PO₂CH₂)Ala-Ala'-NH₂ obtenu dans l'exemple 2, vis-à-vis des mutants N- et C-terminaux de l'ECA. Les résultats obtenus sont reportés dans le tableau I.

D'après ces données, on constate que ce peptide est de trois ordres de grandeur plus actif vis-à-vis du site N-terminal de l'ECA que de son site C-terminal.

### Exemple 4

Dans cet exemple, on synthétise quatre analogues du peptide de l'exemple 3 ayant des groupes terminaux différents et on détermine l'influence des groupes N- et C-terminaux sur la sélectivité vis-à-vis du site N-terminal de l'ECA en utilisant le même mode opératoire que dans l'exemple 1. Les résultats sont donnés dans le tableau II.

Les résultats du tableau II révèlent que la présence du groupe carboxamide est essentielle à la sélectivité de la molécule. En effet, si la présence d'un groupe carboxylate renforce l'affinité vis-à-vis du site N-terminal, cette modification entraîne la perte de sélectivité, ce nouveau composé étant très actif sur le domaine C-terminal. Dans une moindre mesure, on voit que la présence du groupe N-acétyle joue aussi un rôle sur la sélectivité. Le dernier analogue révèle le rôle très important du résidu aspartique en position N-terminale sur la sélectivité.

### Exemple 5

Dans cet exemple, on étudie l'influence de la configuration des résidus Phe et Ala encadrant le bloc phosphinique vis-à-vis de l'affinité du dérivé de peptide de l'exemple 3 pour le site N-terminal. La synthèse du peptide Ac-Asp-Phe-Ψ'PO₂CH₂)Ala-Ala-NH₂ aboutit à l'obtention d'un mélange de quatre diastéréoisomères, dus à la présence de deux centres asymétriques dans ce composé. La purification par chromatographie liquide à haute performance HPLC phase inverse de ce produit sur colonne C18 analytique VYDAC, Spectra System (λ = 220 nm, débit = 1ml/min) permet de séparer trois fractions.

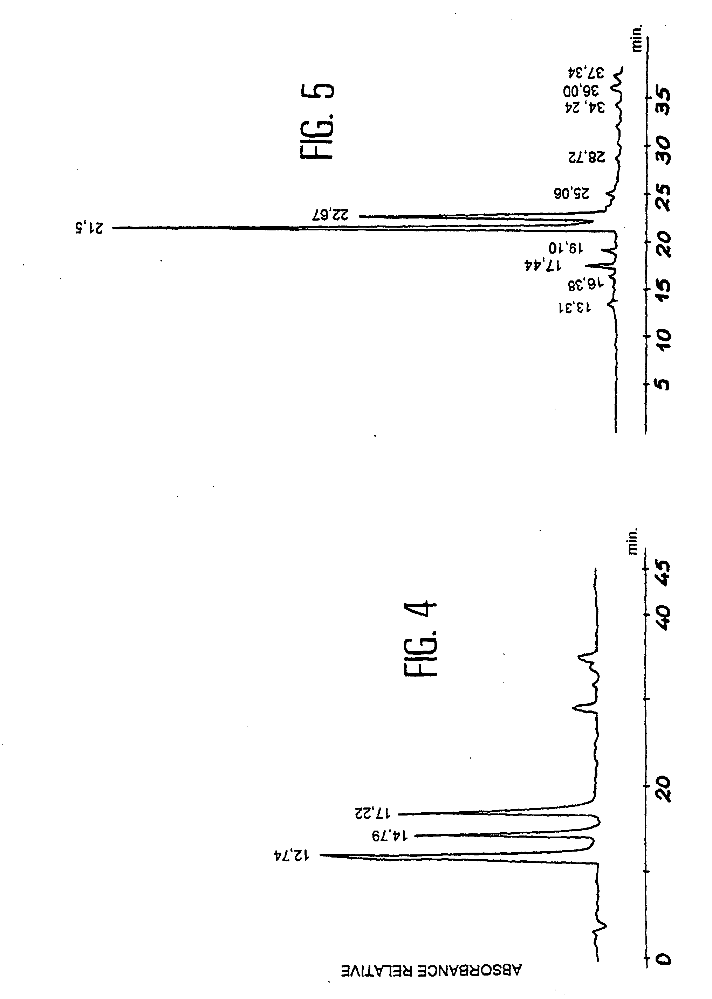

La figure 4 illustre le chromatogramme obtenu qui comprend trois pics

Sur la base de l'intensité des pics HPLC et du spectre RMN du phosphore, on démontre que le premier pic contient deux diastéréoisomères, les deux autres pics ne contenant qu'un seul diastéréoisomère. La détermination de l'activité de ces fractions révèle que seule la première fraction contient des composés actifs.

### Exemple 6

Comme la méthode de purification de l'exemple 5 ne permet pas de séparer les deux diastéréoisomères présents dans la fraction correspondant au premier pic, on effectue une synthèse du peptide Ac-Asp-Phe-Ψ(PO₂CH₂)-Ala-AlaNH₂ à partir d'un résidu phénylaminophosphinique de configuration R afin d'obtenir en fin de synthèse un mélange de deux diastéréoisomères, séparables facilement par HPLC en phase inverse.

Pour réaliser la synthèse du peptide contenant le résidu aminophenylphosphinique de configuration R, on synthétise le racémique de cet acide aminophénylphosphinique, puis on sépare les isomères par recristallisation en présence d'une amine chirale, selon la méthode décrite par Baylis et al., dans J. Chem. Soc. Perkin. Trans (1984) 2845-2849 [15]. Ainsi, on obtient les résidus acide aminophenylphosphinique optiquement purs de configuration R et S. A partir de l'acide de configuration R, on synthétise le bloc Fmoc_{(R)}Phe (PO(Ad)-CH₂)AlaOH comme dans l' exemple 1 pour obtenir un mélange de deux diastéréoisomères

Ac-Asp-_{(R)}Phe(PO₂CH₂)_{(R)}Ala-Ala-NH₂

Ac-Asp-_{(R)}Phe(PO₂CH₂)_{(S)}Ala-Ala-NH₂

que l'on sépare par HPLC en phase inverse C18.

La synthèse du peptide Ac-Asp-PheΨ(PO₂-CH₂)Ala-Ala-NH₂ est effectuée par une stratégie de synthèse en phase solide classique, par chimie Fmoc, en utilisant comme support solide la résine Rink amide. La synthèse e ce peptide est réalisée par le couplage successif des blocs suivants : Fmoc-Ala, Fmoc-Phe(PO(Ad)-CH₂)AlaOH et Fmoc-Asp(t-Bu). Les couplages ont été réalisées par la stratégie in situ utilisant le 2-(1Hbenzotriazol-1-yl)1,1,3,3-tétraméthyluroniumhexafluorophosphate/diisopropyléthylamine. Les conditions de couplage sont les suivantes : trois équivalents de dérivés de Fmoc et 4 équivalents de diisopropyléthylamine dans le diméthylformamide sont ajoutés à la résine et laissés réagir pendant 30 minutes. Pour cliver le groupe Fmoc, les conditions suivantes ont été utilisées : 50 % de pipéridine dans le diméthylformamide, pendant 30 minutes. Le clivage du peptide de la résine, ainsi que des groupements protecteurs sont effectués par traitement avec une solution d'acide trifluroacétique contenant 2,5 % d'eau, 2,5 % de thioanisol, 2/5 % de phénol, 1,25 % d'éthanedithiol et 1,25 % de triisopropylsilane. La purification du produit a été réalisé par HPLC phase inverse C-18.

La figure 5 illustre le chromatogramme obtenu dans ces conditions qui comprend deux pics, dont un seul, le premier, possède un pouvoir inhibiteur sur l'ECA. Le pic actif contient une molécule se caractérisant par un Ki de 12 nM sur le site N-terminal et par un Ki de 25 µM sur le site C-terminal de l'ECA.

La fraction la plus active du peptide contient un résidu pseudo-alanine de configuration S. La structure active du peptide est donc du type : Ac-Asp-_{(R)}Phe(Po₂CH₂)_{(S)}Ala-Ala-NH₂ (III), dénommé ci-après RXP 407. Les constantes d'inhibition de ce composé optiquement pur sont reportées dans le tableau III.

La pureté du composé RXP 407 a pu être établie par spectrométrie de masse (masse théorique 498,5, masse exp 498), et par résonance magnétique du phosphore, du proton et du carbone 13.

Spectre phosphore : d= 40,78 ppm, référence acide phosphorique (0 ppm)

Spectre proton (ppm): Asp: Ha, 4,28, Hb,b', 2,0 et 2,28; Phe: Ha, 4,08, Hb,b', 2,55 et 3,10, Ar, 7,13 et 7,22; Ala: Ha, 2,58, Hb, 1,08; Ala: Ha, 4,12, Hb, 1,28; P-CH2, 1,48 et 1,62; CH3-CO, 1,82. référence TSP (0 ppm).

Spectre carbone 13 (ppm): Asp: Ca, 55,3, Cb, 41,5, CO, 180,5; Phe: Ca, 54,5, Cb, 36, Ar, 129,8, 131,7, 132,5, 141,2; Ala: Ca, 37,6, Cb, 21,4, CO, 182,3; Ala: Ca, 53, Cb, 19,6, CO, 181,5; CH2-P, 34,4; CH3-CO, 24,5 et 177. référence TSP (0 ppm).

Les spectres ont été déterminés dans D₂O, sur un appareil du type Bruker DRX, opérant à la fréquence de 250MHz pour le proton, de 101 MHz pour le phosphore et de 62 MHz pour le carbone. Les attributions ont été réalisées à l'aide d'expériences bidimensionnelles de type COSY, TOCSY, HMQC et HMBC.

Le composé RXP 407 est très intéressant car il est capable de discriminer les deux sites actifs de L'ECA. Ce composé est un inhibiteur puissant du site N-terminal de l'ECA (Ki = 12 nM), mais a très peu d'affinité pour le site C-terminal (Ki = 25 µM). Outre cette propriété, on peut remarquer que cette molécule, compte tenu des résidus Asp et pseudo-Phe, implique lors de son interaction les sous sites S₁ et S₂ de l'ECA, une propriété peu usuelle pour un inhibiteur d'ECA. Encore plus surprenant, cette molécule comporte en position C-terminale de sa structure, un groupe carboxamide, alors que tous les inhibiteurs de l'ECA décrits à ce jour possèdent systématiquement un groupe carboxylate libre. Ainsi, cette molécule apparaît très originale tant du point de vue de sa structure chimique que de son activité inhibitrice.

### Exemple 7

Dans cet exemple, on utilise le peptide RXP 407 comme inhibiteur de l'ECA naturelle, et on étudie l'influence de la concentration en RXP 407 (en nM) sur le pourcentage d'inhibition de l'ECA.

Le pouvoir inhibiteur du composé est déterminé comme dans l'exemple 1 en utilisant l'ECA sauvage humaine produite par des cellules CHO selon le protocole décrit dans la référence Wei et al, J. Biol. Chem. (1992) 267, 13398-13405 [14]

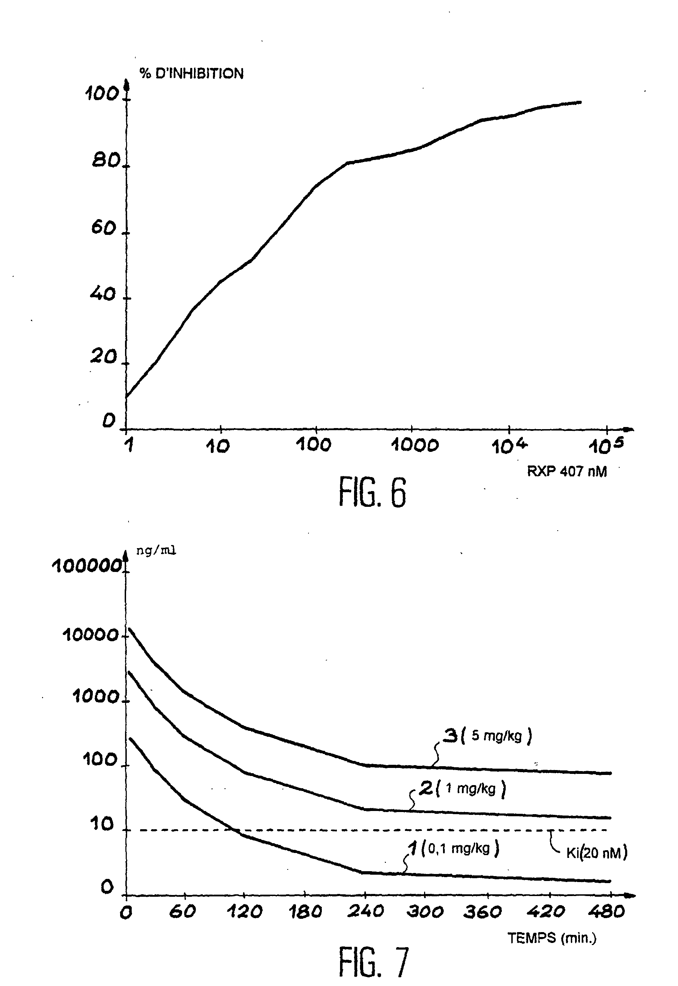

Les résultats obtenus sont représentés sur la figure 6 qui illustre le profil d'inhibition que l'on peut obtient avec le RXP 407 pour l'inhibition de l'ECA naturelle. La présence de deux paliers dans la courbe d'inhibition traduit l'existence des deux sites actifs dans l'ECA naturelle, la partie gauche de la courbe étant due à l'inhibition du domaine N-terminal de l'ECA, la partie droite étant due à celle du domaine C-terminal. Les points d'inflexion apparaissant sur cette courbe indiquent des valeurs IC50 du RXP 407 vis-à-vis des sites N et C-terminaux de l'ECA naturelle qui sont compatibles avec les valeurs de Ki mesurées à partir des mutants de l'ECA.

Cette expérience démontre que le RXP 407 agit bien comme un inhibiteur sélectif du domaine N-terminal de l'enzyme naturelle. Il est intéressant de remarquer que la forme de la courbe est due au fait que le substrat utilisé dans cette expérience est aussi bien clivé par le domaine N-terminal que C-terminal.

Ainsi, il est possible d'utiliser le RXP 407 pour identifier des substrats qui seraient clivés par un seul des deux sites actifs de l'enzyme. En effet, pour un substrat uniquement clivé par l'un des deux sites, la courbe d'inhibition ne contiendra qu'un seul palier et non pas deux. Si le substrat est uniquement clivé par le domaine N-terminal, le point d'inflexion de la courbe sera situé vers la concentration de RXP 407 de 100 nM, alors que pour un substrat uniquement clivé par le domaine C-terminal, le point d'inflexion sera déplacé vers 10 mM.

Ainsi, ce nouvel inhibiteur est un très bon outil pour étudier la sélectivité de l'ECA naturelle vis-à-vis de la dégradation de substrats physiologiques.

### Exemple 8

Dans cet exemple, on étudie la pharmacocinétique et le métabolisme du composé RXP 407 chez l'animal.

Dans ce but, on réalise la synthèse d'un RXP 407 comportant trois atomes de tritium sur le groupe N-acétyle afin de pouvoir entreprendre une étude sur la pharmacocinétique et le métabolisme de cette molécule chez le rat, en injectant ce produit à des doses de 0,1, 1 et 5 mg/kg par voie intraveineuse en bolus.

On détermine ensuite la concentration plasmatique de RXP 407 en fonction du temps.

Les résultats obtenus sont représentés sur la figure 7. La courbe 1 correspond à 0,1 mg/kg, la courbe 2 à 1 mg/kg et la courbe 3 à 5 mg/kg. La courbe en pointillé illustre le Ki du RXP 407 (20 nM).

Ainsi, on remarque que l'injection d'une dose de 5 mg/kilo par intraveineuse en bolus aboutit à des concentrations plasmatiques de RXP 407 de 100 ng/ml de produit pendant plus de quatre heures, ce qui correspond à une concentration égale à 10 fois le Ki de cet inhibiteur pour le site N-terminal.

Dans le tableau IV, on a reporté les résultats obtenus en ce qui concerne l'élimination du RXP 407 tritié in vivo chez le rat. Ces résultats sont exprimés en % de la radioactivité injectée. Ils montrent que le composé est majoritairement éliminé dans les urines ( 87 % après 24h), et très peu dans les fécès (13%). Aucune trace de ce produit n'est retrouvée dans les voies aériennes.

D'après ce tableau on voit que les 4 rats ont éliminé 100% (100,09 ± 7) du RXP 407 injecté en 48 heures. Le produit est éliminé majoritairement dans les urines, minoritairement dans les fécès, pratiquement pas par les voies aériennes.

Par ailleurs, l'analyse de la structure du produit dans les urines et les fécès démontre que le RXP 407 ne subit aucun métabolisme.

On a évalué la toxicité de ce produit chez la souris. On a ainsi vérifié qu'à une dose de 25mg/kg, injecté par voie intraveineuse, ce produit n'entraîne aucun signe de toxicité chez la souris. Après 7 jours de surveillance, les souris traitées vivent tout à fait normalement.

Ainsi, le RXP 407 est un inhibiteur efficace. L'intérêt de ce nouvel inhibiteur de l'ECA réside dans le fait qu'il représente le premier inhibiteur sélectif du site N-terminal de cette enzyme. Les propriétés pharmacocinétiques, l'absence de métabolisme et donc la stabilité in vivo font de cet inhibiteur un produit idéal pour, dans des protocoles thérapeutiques, bloquer le site N-terminal de l'ECA sans affecter les fonctions physiologiques qui seraient contrôlées par le site C-terminal de l'enzyme.

Par ailleurs, le RXP 407, permet d'établir in vivo la contribution respective des sites N-terminal et C-terminal de l'ECA dans le métabolisme de l'Ac-Ser-Asp-Lys-Pro. Les expériences menées in vitro démontrent que le site N-terminal de l'ECA est beaucoup plus efficace pour cliver ce peptide que le site C-terminal, le contrôle du métabolisme de ce peptide via le RXP 407 est donc un objectif raisonnable.

### Exemple 9

Dans cet exemple, on étudie l'effet de l'injection in vivo de RPX 407 chez des souris.

Dans ce but, on soumet les souris à une perfusion de RXP 407 pendant 30 minutes, à des doses de 0,1, 1 et 10 mg/kg et on détermine la concentration en peptide Ac-Ser-Asp-Lys-Pro-(Ac-SDKP) du plasma des souris.

L'Ac-Ser-Asp-Lys-Pro est déterminé par un dosage immunoenzymatique par compétition utilisant des anticorps polyclonaux et de l'Ac-SDKP lié à l'acétylcholinestérase d'Electrophorus Electricus.

On effectue les mêmes mesures de concentrations plasmatiques en Ac-SDKP sur des souris perfusées avec du Lisinopril à une dose de 10 mg/kg et sur des souris témoins non perfusées. Le Lisinopril est un inhibiteur utilisé en clinique qui bloque de façon non sélective les deux sites actifs de l'ECA.

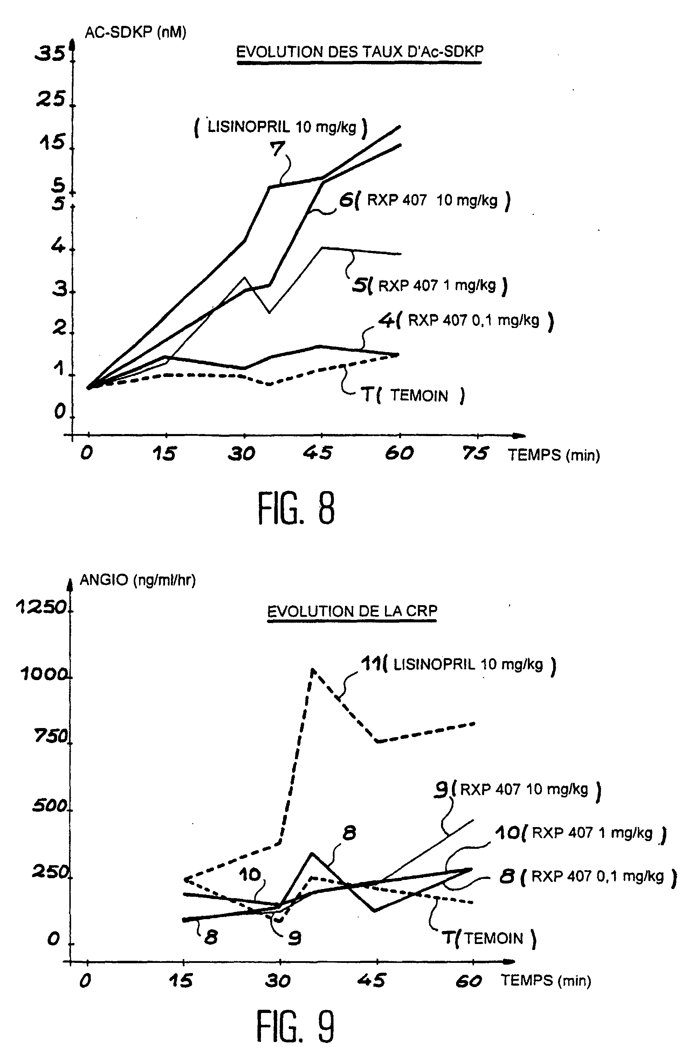

La figure 8 illustre les résultats obtenus, soit la concentration en Ac-SDKP (nM) en fonction du temps (en minute).

Les courbes 4, 5 et 6 se rapportent aux souris perfusées avec RXP 407 aux doses de 0,1 mg/kg (courbe 4), 1 mg/kg (courbe 5) et 10 mg/kg (courbe 6).

La courbe 7 se rapporte aux souris perfusées avec 10 mg/kg de Lisinopril.

La courbe T se rapporte aux souris témoins. On constate que l'injection du RXP 407 provoque une augmentation très importante du taux plasmatique du peptide Ac-SDKP. Par rapport à un inhibiteur bloquant de façon non sélective les deux sites actifs de l'ECA, on remarque que les taux de Ac-SDKP dans le plasma sont les mêmes que ceux obtenus avec le RXP 407. Sachant que le RXP 407 ne bloque qu'un seul des deux sites de l'ECA, le site N-terminal, on peut conclure de ces expériences : que le site C-terminal de l'ECA n'est pas impliqué dans le métabolisme de Ac-SDKP et que par contre ce métabolisme ne dépend que du site N-terminal de l'ECA, d'où l'intérêt de l'utilisation du RXP 407, par rapport à des inhibiteurs classiques de l'ECA, qui bloquent les deux sites de l'ECA de façon non sélective. Cette expérience démontre aussi l'activité in vivo effective du RXP 407.

### Exemple 10

Dans cet exemple, on détermine l'effet de RXP 407 sur les taux de la rénine, une autre enzyme intervenant dans la régulation de la pression artérielle. Le blocage de l'ECA par un inhibiteur classique de cette enzyme entraîne l'arrêt de l'hydrolyse de l'angiotensine I en angiotensine II. L'angiotensine I est en effet un des substrats physiologiques de l'ECA. La diminution du taux d'angiotensine Il dans le plasma, par un mécanisme de rétrocontrôle, augmente le taux plasmatique de la rénine, enzyme qui intervient directement dans la production d'angiotensine I, à partir de l'angiotensinogène.

Ainsi, l'inhibition de l'ECA peut être suivie de façon indirecte par le taux plasmatique de rénine.

Ce taux peut être évalué par la capacité de la rénine dans des échantillons de plasma d'hydrolyser un excès d'angiotensinogène présent dans le plasma de souris. La formation d'angiotensine I est alors mesurée par un dosage radioimmunologique et le taux de rénine est exprimé en ng d'angiotensine I formée par ml de plasma et par h d'incubation.

Les résultats obtenus sont donnés sur la figure 9 pour :
- des souris perfusées avec RXP 407 à des doses de 0,1 mg/kg (courbe 8), de 1 mg/kg (courbe 9) et de 10 mg/kg (coube 10),
- des souris perfusées avec du Linisopril à une dose de 10 mg/kg(courbe 11), et
- des souris témoins (courbe T).

La figure 9 montre que le traitement d'une souris avec le Lisinopril entraîne l'augmentation plasmatique du taux de rénine, ce qui est conforme à l'effet d'inhibition de l'ECA par ce composé. Par contre, on montre que le traitement par RXP 407 n'entraîne pas d'augmentation de rénine. Ainsi, cet inhibiteur permet d'inhiber sélectivement la dégradation du peptide Ac-SDKP, sans affecter la régulation de la pression artérielle. Cette observation, très importante quant à la sélectivité de l'activité in vivo du composé RXP 407, suggère que seul le site C-terminal de l'ECA intervient dans l'hydrolyse de l'angiotensine I, expliquant ainsi pourquoi l'injection de RXP 407 n'a pas d'influence sur les taux de rénine.

Ainsi, l'ensemble de ces expériences confirme l'intérêt de l'utilisation du RXP 407 pour contrôler les taux plasmatiques du peptide Ac-SDKP. Pour rappel, il a été démontré que ce peptide avait un effet protecteur sur les cellules souches de la moelle osseuse lors des protocoles de chimio ou de radiothérapie. Le RXP 407 sera donc très utile dans ce contexte, par mobilisation des taux physiologiques du peptide Ac-SDKP, via le bloquage de son métabolisme par l'ECA.

Le peptide Ac-Ser-Asp-Lys-Pro étant un régulateur de l'hématopoïèse, on peut proposer d'utiliser l'inhibiteur de l'invention dans des protocoles de chimiothérapies ou de radiothérapies anti-cancéreuses, afin de protéger les cellules de la moelle osseuse des effets toxiques du traitement anti-cancéreux. Il est possible d'envisager aussi une application dans le domaine de la thérapeutique anti-cancéreuse puisqu'il reste envisageable que l'ECA intervienne dans la prolifération de différents types cellulaires via l'activité de son domaine N-terminal.

Une autre retombée concernant cet inhibiteur est qu'il représente un outil très efficace pour étudier la contribution respective des sites N- et C-terminaux de l'ECA sauvage dans la dégradation de différents substrats physiologiques de cette enzyme.

### Références citées

[1] : Soubrier et al, **Proc. Natl. Acad. Sci.** USA (1988) 85, 9386-9390.
[2] : Lenfant et al, **Proc. Natl. Acad. Sci**. USA (1989) 86, 779-782.
[3] : Lombard et al,**Cell. Tissue. Kinet** (1990) 23, 99-103.
[4] : Bodgen et al, **Ann. N.Y. Acad. Sci**. (1991) 628, 126-139.
[5] Rousseau et al, **J. Biol. Chem.** (1995) 270, 3656-3661.
[6] : Azizi et al, **J. Clin. Invest.** (1996) 97, 839-844.
[7] : Volpert et al, **J. Clin. Invest**. (1996) 98, 671-679.
[8] : FR-A-2 676 059.
[9] : EP-A-0 725 075.
[10] : Yiotakis et al, **J. Org. Chem** (1996) 61, 6601-6605.
[11] : Barlos étal, **G. Int. J. Pept. Protein. Res**. 1991, 37, 513-520.
[12] : Jiracek et al, **J. Biol. Chem.** (1995) 270, 21701-21706.
[13] : Jiracek Yiotakis et al, **J. Biol Chem.** (1996) 271, 19606-19661.
[14] : Wei et al, **J. Biol. Chem.** (1992) 267, 13398-13405.
[15] : Baylis et al., **J. Crieur. Soc. Perkin. Trans** (1984) 2845-2849.

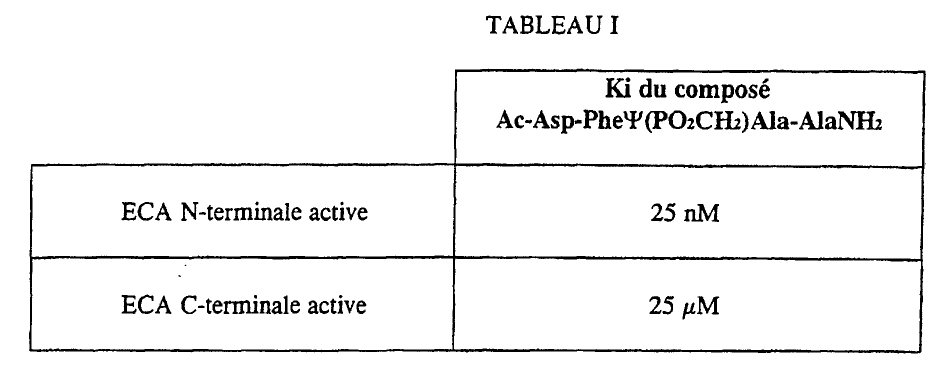

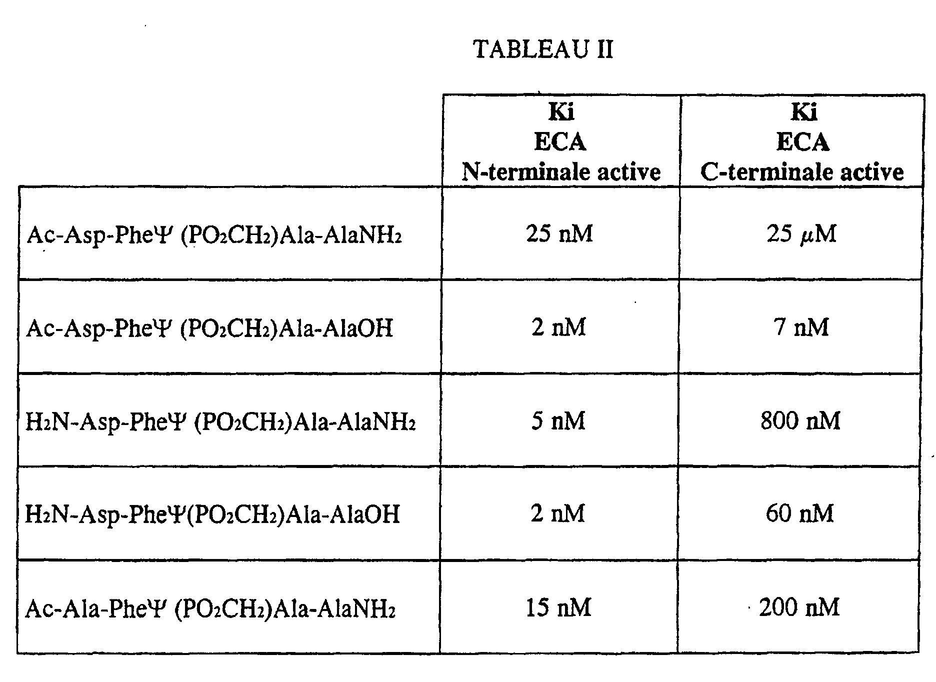

**TABLEAU III**

| | **Ki ECA N-terminale active** | **Ki ECA C-terminale active** |
|---|---|---|
| Ac-Asp-_{(R)}PheΨ (PO₂CH₂)_{(S)}Ala-AlalNH₂ | 12 nM | 25 µM |

**TABLEAU IV:**

| (% de la radioactivité injectée) | | | | | | | | | | | |
|---|---|---|---|---|---|---|---|---|---|---|---|
| | **Urines** | | | | **Fècès** | | **Air expiré** | | | | |
| Rat | 4 h | 8 h | 24 h | 48h | 24 h | 48 h | 4 h | 8 h | 24 h | 48 h | Total |
| 1 | 0 | 89,79 | 6,71 | 0,59 | 2,70 | 1,83 | 0 | 0 | 0,01 | 0 | 101,80 |
| 2 | 45,14 | 20,77 | 4,24 | 1,23 | 6,69 | 19,32 | 0 | 0 | 0,02 | 0 | 98,86 |
| 3 | 68,84 | 6,30 | 2,19 | 0,87 | 6,02 | 4,94 | 0 | 0 | 0,02 | 0,01 | 93,20 |
| 4 | 0 | 81,27 | 5,19 | 0,86 | 8,58 | 3,93 | 0 | 0 | 0,01 | 0 | 106,49 |
| Moyenne de 4 rats | 28,49 | 49,53 | 4,58 | 0,89 | 6,00 | 7,51 | 0 | 0 | 0,02 | 0 | 100,09 ± 7 |

## Revendications

1. Dérivé de peptide comportant la séquence d'acides aminés de formule suivante :
-Asp-Phe-Ψ(PO₂CH₂)-Ala-Xaa'- (I)
dans laquelle :
- Ψ(PO₂CH₂) indique que la liaison peptidique (CONH) entre Phe et Ala a été remplacée par la liaison phosphinique PO₂CH₂, et
- Xaa' représente un résidu d'acide aminé, le groupe PO₂CH₂ étant sous forme PO₂⁻ et associé à un contre-ion acceptable du point de vue pharmaceutique.

2. Dérivé de peptide répondant à la formule suivante :
R¹-Asp-Phe-Ψ(PO₂CH₂)-Ala-Xaa'-NH₂ (II)
dans laquelle :
- R¹ représente le groupe acétyle ou benzyloxycarbonyle,
- Ψ(PO₂CH₂) indique que la liaison peptidique (CONH) entre Phe et Ala a été remplacée par la liaison phosphinique PO₂CH₂, et
- Xaa' représente un résidu d'acide aminé, le groupe PO₂CH₂ étant sous forme PO₂⁻ et associé à un contre-ion acceptable du point de vue pharmaceutique.

3. Dérivé de peptide selon la revendication 2, dans lequel R¹ représente le groupe acétyle.

4. Dérivé de peptide selon l'une quelconque des revendications 1 à 3, dans lequel Xaa' représente Ala.

5. Dérivé de peptide selon l'une quelconque des revendications 1 à 4, dans lequel le résidu Phe à la configuration R.

6. Dérivé de peptide selon l'une quelconque des revendications 1 et 2, dans lequel le résidu Ala a la configuration S.

7. Dérivé de peptide de formule :
Ac-Asp-_{(R)}Phe-Ψ(PO₂CH₂)-_{(S)}Ala-Ala-NH₂ (III)
dans laquelle :
- Ac représente le groupe acétyle, et
- Ψ(PO₂CH₂) indique que la liaison peptidique (CONH) entre Phe et Ala a été remplacée par la liaison phosphinique (PO₂CH₂), le groupe PO₂CH₂ étant sous forme PO₂⁻ et associé à un contre-ion acceptable du point de vue pharmaceutique.

8. Composition pharmaceutique inhibant sélectivement le site N-terminal de l'enzyme de conversion de l'angiotensine humaine, comprenant un dérivé de peptide selon l'une quelconque des revendications 1 à 7.

9. Composition pharmaceutique selon la revendication 8, utilisable pour protéger les cellules souches hématopoïétiques de patients soumis à un traitement chimiothérapique ou radiothérapique agressif.

10. Composition selon la revendication 8, dans laquelle le traitement est un traitement anti-cancéreux.

11. Utilisation d'un dérivé de peptide selon l'une quelconque des revendications 1 à 7 pour la fabrication d'un médicament inhibant sélectivement le site N-terminal de l'enzyme de conversion de l'angiotensin humaine.

12. Utilisation selon la revendication 11, dans laquelle le médicament est destiné à réguler la prolifération des cellules souches hématopoïétiques de patients soumis à un traitement anti-cancéreux.

## Claims

1. Peptide derivative comprising the amino acid sequence according to the following formula:
-Asp-Phe-Ψ(PO₂CH₂)-Ala-Xaa'- (I)
wherein:
- Ψ(PO₂CH₂) indicates that the peptide bond (CONH) between Phe and Ala has been replaced by the phosphonic bond PO₂CH₂, and
- Xaa' represents an amino acid residue, the group PO₂CH₂ being in the form PO-₂ and associated with a pharmaceutically acceptable counterion.

2. Peptide derivative observing the following formula:
R¹-Asp-Phe-Ψ(PO₂CH₂)-Ala-Xaa'-NH₂ (II)
wherein:
- R¹ represents the acetyl or benzyloxycarbonyl group,
- Ψ(PO₂CH₂) indicates that the peptide bond (CONH) between Phe and Ala has been replaced by the phosphonic bond PO₂CH₂, and
- Xaa' represents an amino acid residue, the group PO₂CH₂ being in the form PO-₂ and associated with a pharmaceutically acceptable counterion.

3. Peptide derivative according to claim 2, wherein R¹ represents the acetyl group.

4. Peptide derivative according to any of claims 1 to 3, wherein Xaa' represents Ala.

5. Peptide derivative according to any of claims 1 to 4, wherein the Phe residue has an R configuration.

6. Peptide derivative according to any of claims 1 and 2, wherein the Ala residue has an S configuration.

7. Peptide derivative according to the formula:
Ac-Asp-_{(R)}Phe-Ψ(PO₂CH₂)-_{(S)}Ala-Ala-NH₂ (III)
wherein:
- Ac represents the acetyl group,
- Ψ(PO₂CH₂) indicates that the peptide bond (CONH) between Phe and Ala has been replaced by the phosphonic bond (PO₂CH₂), the group PO₂CH₂ being in the form PO-₂ and associated with a pharmaceutically acceptable counterion.

8. Pharmaceutical formulation selectively inhibiting the N-terminal site of human angiotensin-converting enzyme, comprising a peptide derivative according to any of claims 1 to 7.

9. Pharmaceutical formulation according to claim 8, that can be used to protect haematopoietic strain cells of patients undergoing aggressive chemotherapy or radiotherapy treatment.

10. Formulation according to claim 8, wherein the treatment is a cancer treatment.

11. Use of a peptide derivative according to any of claims 1 to 7 to produce a medicinal product selectively inhibiting the N-terminal site of human angiotensin-converting enzyme.

12. Use according to claim 11, wherein the medicinal product is intended to regulate the proliferation of haematopoietic strain cells of patients undergoing cancer treatment.

## Patentansprüche

1. Peptid-Derivat, das die folgende Aminosäuresequenz aufweist:
-Asp-Phe-Ψ(PO₂CH₂)-Ala-Xaa'- (I)
worin:
- Ψ(PO₂CH₂) anzeigt, dass die Peptid-Bindung (CONH) zwischen Ph und Ala durch die Phosphin-Bindung PO₂CH₂ ersetzt ist, und
- Xaa' einen Aminosäurerest darstellt,
wobei die PO₂CH₂-Gruppe in der Form PO₂⁻ vorliegt und mit einem pharmazeutisch akzeptablen Gegenion assoziiert ist.

2. Peptid-Derivat der folgenden Formel:
R¹-Asp-Ph-Ψ(PO₂CH₂)-Ala-Xaa'-NH₂ (II)
worin:
- R¹ die Acetyl- oder Benzyloxycarbonyl-Gruppe darstellt,
- Ψ(PO₂CH₂) anzeigt, dass die Peptid-Bindung (CONH) zwischen Phe und Ala durch die Phosphin-Bindung PO₂CH₂ ersetzt ist, und
- Xaa' für einen Aminosäurerest steht,
wobei die PO₂CH₂-Gruppe in der Form PO₂⁻ vorliegt und mit einem pharmazeutisch akzeptablen Gegenion assoziiert ist.

3. Peptid-Derivat nach Anspruch 2, in dem R¹ die Acetylgruppe darstellt.

4. Peptid-Derivat nach einem der Ansprüche 1 bis 3, in dem Xaa' Ala darstellt.

5. Peptid-Derivat nach einem der Ansprüche 1 bis 4, in dem der Rest Phe die R-Konfiguration hat.

6. Peptid-Derivat nach einem der Ansprüche 1 und 2, in dem der Rest Ala die S-Konfiguration hat.

7. Peptid-Derivat der Formel:
Ac-Asp-_{(R)}Phe-Ψ(PO₂CH₂)-_{(S)}Ala-Ala-NH₂ (III)
worin:
- Ac die Acetylgruppe darstellt und
- Ψ(PO₂CH₂) anzeigt, dass die Peptid-Bindung (CONH) zwischen Phe und Ala durch die Phosphin-Bindung (PO₂CH₂) ersetzt ist,
wobei die PO₂CH₂-Gruppe in der Form PO₂⁻ vorliegt und mit einem pharmazeutisch akzeptablen Gegenion assoziiert ist.

8. Pharmazeutische Zusammensetzung zur selektiven Hemmung der N-terminalen Stelle des humanen Anglotensin-Umwandlungsenzyms, die ein Peptid-Derivat nach einem der Ansprüche 1 bis 7 enthält.

9. Pharmazeutische Zusammensetzung nach Anspruch 8, die verwendbar ist zum Schützen der hämatopoietischen Stammzellen von Patienten, die einer aggressiven chemotherapeutischen oder radiotherapeutschen Behandlung unterzogen werden.

10. Zusammensetzung nach Anspruch 8, in der die Behandlung eine Antikrebs-Behandlung ist.

11. Verwendung eines Peptid-Derivats nach einem der Ansprüche 1 bis 7 zur Herstellung eines Arzneimittels zur selektiven Hemmung der N-terminalen Stelle des humanen Anglotensin-Umwandlungsenzyms.

12. Verwendung nach Anspruch 11, bei der das Arzneimittel dazu bestimmt ist, die Vermehrung der hämatopoietischen Stammzellen von Patienten, die einer Antikrebsbehandlung unterzogen werden, zu steuern.
